(19) Europäisches Patentamt
European Patent Office
Office européen des brevets

(11) Veröffentlichungsnummer: **0 495 175 B1**

(12) **EUROPÄISCHE PATENTSCHRIFT**

(45) Veröffentlichungstag der Patentschrift: **22.03.95**

(51) Int. Cl.[6]: **C07C 69/608**, C07C 67/38

(21) Anmeldenummer: **91119601.2**

(22) Anmeldetag: **16.11.91**

(54) **Carboxylgruppenhaltige Cyclohexanderivate.**

(30) Priorität: **18.01.91 DE 4101355**

(43) Veröffentlichungstag der Anmeldung:
**22.07.92 Patentblatt 92/30**

(45) Bekanntmachung des Hinweises auf die Patenterteilung:
**22.03.95 Patentblatt 95/12**

(84) Benannte Vertragsstaaten:
**DE FR GB NL**

(56) Entgegenhaltungen:
**EP-A- 0 400 292**
**FR-A- 1 567 495**
**GB-A- 858 257**

(73) Patentinhaber: **HÜLS AKTIENGESELLSCHAFT**
**D-45764 Marl (DE)**

(72) Erfinder: **Schröder, Wolfgang, Dr.**
**Föckerskamp 3**
**W-4270 Dorsten (DE)**
Erfinder: **Keil, Thomas, Dr.**
**Siegerlandstrasse 27**
**W-4350 Recklinghausen (DE)**

EP 0 495 175 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die neuen carboxylgruppenhaltigen Cyclohexanderivate der allgemeinen Struktur

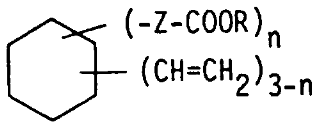

(I)

alleine oder im Gemisch miteinander, ihre Herstellung sowie ihre Verwendung. Bei den Cyclohexanderivaten der Struktur I stehen die Substituenten in 1-, 2- und 4-Position, ist Z eine $-CH_2-CH_2-$Gruppe (Ethylengruppe) oder eine

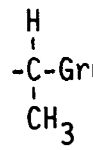-Gruppe

(Ethylidengruppe), R eine Alkylgruppe mit 1 bis 10 C-Atomen und n eine Zahl von 1 bis 3.

Carboxylgruppenhaltige Cyclohexanderivate sind als Monomere und Comonomere bei der Herstellung von Polymeren von großem Interesse.

1,2,4-Trivinylcyclohexan, das bei dem erfindungsgemäßen Verfahren als Ausgangsverbindung eingesetzt wird, kann beispielsweise nach R. Rienäcker, Brennstoff-Chemie 45, 206 (1964) durch Pyrolyse von 1,5,9-Cyclododecatrien erhalten werden.

Nach DE-B-29 12 489 können Monoolefine mit innenständiger Doppelbindung in Gegenwart einer Kobaltverbindung als Katalysator und von Pyridin oder eines nicht ortho-alkylierten Pyridins als Promotor bei 165 bis 195 °C und einem Druck von 150 000 bis 300 000 hPa hydrocarboxyalkyliert werden. Ein Hinweis auf das Verhalten von Dienen oder Polyenen unter diesen Bedingungen wird in der Schrift nicht gegeben.

J. Falbe, Synthesen mit Kohlenmonoxid, Berlin - Heidelberg - New York, 1967, gibt an, daß 1,5-Cyclooctadien an einem Palladiumkatalysator entweder zu einem ungesättigten Monocarbonsäureester oder zu einem gesättigten Dicarbonsäureester hydrocarboxyalkyliert wird. Bei der analogen Hydrocarboxylierung von 1,5-Cyclooctadien an einem Kobaltkatalysator erhält man dagegen nur eine gesättigte Monocarbonsäure. Am Kobaltkatalysator wird also von den zwei innenständigen Doppelbindungen eine hydrocarboxyliert und eine hydriert.

Laut U. Buller (Dissertation, Rheinisch-Westfälische Technische Hochschule Aachen, 1980) reagieren alpha,omega-Diene mit Kohlenmonoxid und einem Alkohol bei 130 bis 140 °C in hoher Selektivität zu Dicarbonsäureestern.

In DE-A-38 12 184 werden reaktive alpha,omega-Diene an Kobaltkatalysatoren hydrocarboxyalkyliert. Dabei erhält man bei 100 bis 200 °C und 150 000 bis 350 000 hPa omega-en-Carbonsäureester. Es wird also nur eine der endständigen Doppelbindungen hydrocarboxyalkyliert. Eine Doppelbindung bleibt hier erhalten. Die Umsätze der alpha,omega-Diene liegen jedoch in den Beispielen immer unter 50 %.

GB-A-858 257 betrifft spezielle Carbonsäureanhydride, die aus Maleinsäureanhydrid und u. a. Trivinylcyclohexan durch thermische Addition erhalten werden. Dabei können die Verbindungen, die neben Anhydridgruppen auch nicht umgesetzte Doppelbindungen enthalten, mit weiteren Verbindungen zu Polymeren umgesetzt werden. Die dabei erhaltenen Produkte weisen Doppelbindungen auf und stehen weiteren Modifikationen zur Verfügung.

In FR-A-1 567 495 wird die Herstellung von Polymeren mit Dimerfettsäuren beschrieben. Für die Synthese der Dimerfettsäuren werden dabei Cyclohexene, die 4 Substituenten mit zum Teil Doppelbindungen aufweisen, als Zwischenprodukte angegeben.

Nach EP-A-0 400 292 können Cyclooctandicarbonsäurediester durch Hydrocarboxyalkylierung von 1.5-Cyclooctadien hergetellt werden. Die Reaktion erfolgt hier in Gegenwart von Kobaltkatalysatoren und tertiären Aminen.

Danach war es noch nicht bekannt, daß endständige Triene hydrocarboxyalkyliert werden können. Es war auch nicht bekannt, zu welchen Produkten eine Hydrocarboxyalkylierung von derartigen Trienen führen würde.

Aufgabe der vorliegenden Erfindung war es, carboxylgruppenhaltige Cyclohexanderivate der allgemeinen Struktur I einzeln und im Gemisch miteinander herzustellen.

Die Aufgabe wird erfindungsgemäß dadurch gelöst, daß man 1,2,4-Trivinylcyclohexan mit einem Alkohol mit 1 bis 10 C-Atomen und Kohlenmonoxid in Gegenwart eines Kobaltkatalysators und eines tertiären Amins bei 120 bis 170 °C und einem Druck von 150 000 bis 350 000 hPa in 6 bis 48 Stunden umsetzt.

Die bei der Reaktion anfallende Mischung enthält die genannten Verbindungen in der Regel nicht in etwa gleichen Mengen. Die Reaktion liefert vielmehr einige Verbindungen als Hauptprodukte, während andere als Nebenprodukte oder nur in Spuren gebildet werden.

Aus dem Reaktionsgemisch können die Verbindungen einzeln isoliert werden. Das kann nach bekannten Methoden, wie Chromatographie oder fraktionierte Destillation, erfolgen.

Die einzelnen Verbindungen und auch die Mischungen sind Gegenstand dieser Erfindung.

Bei der Hydrocarboxyalkylierung verwendet man Alkohole mit 1 bis 10 C-Atomen. Beispiele dafür sind Methanol, Ethanol, n- und iso-Propanol, n- und iso-Butanol, Hexanol, 2-Ethylhexanol und n-Octanol. Alkohole mit 1 bis 4 C-Atomen werden dabei bevorzugt eingesetzt, wobei man Methanol ganz besonders bevorzugt. Der Alkohol ist bei der Reaktion Reagenz und Lösemittel.

Vorzugsweise setzt man 1,2,4-Trivinylcyclohexan und Alkohol im Molverhältnis 1 : 1 bis 1 : 10 ein.

Eine geeignete Kobaltverbindung zur katalytischen Hydrocarboxyalkylierung von 1,2,4-Trivinylcyclohexan ist Hydridokobalttetracarbonyl [$HCo(CO)_4$]. Es können aber auch Kobaltsalze, wie Kobalt(II)acetat, -naphthenat, -stearat, -carbonat oder -chlorid, Kobaltoxide oder Kobaltkomplexe, wie Dikobaltoctacarbonyl, eingesetzt werden. Diese Kobaltverbindungen erfordern in der Anfangsphase der Hydrocarboxyalkylierung neben Kohlenmonoxid 0,1 bis 10 Mol-% Wasserstoff, bezogen auf Mole Kohlenmonoxid. Dabei wird unter den gegebenen Reaktionsbedingungen Hydridokobalttetracarbonyl gebildet, das vermutlich die eigentlich katalytisch aktive Verbindung ist.

Auch beim Einsatz von Hydridokobalttetracarbonyl ist ein kleiner Anfangsgehalt an Wasserstoff hilfreich, da Wasserstoff verbrauchten Katalysator regeneriert.

Bezogen auf eingesetztes 1,2,4-Trivinylcyclohexan verwendet man vorzugsweise 0,5 bis 10 Mol-% Kobaltverbindung.

Als tertiäres Amin, das als Promotor fungiert, kommen Pyridin und nicht ortho-alkylierte Pyridine, wie z. B 3- und 4-Picolin, 3,4- und 3,5-Lutidin, 3- und 4- Ethylpyridin oder andere 3- und 4-Alkylpyridine, wobei Alkyl Propyl, i-Propyl, Butyl, i-Butyl oder t-Butyl sein kann, infrage. Vorzugsweise werden nicht ortho-alkylierte Pyridine eingesetzt, wobei man 4-Picolin ganz besonders bevorzugt.

Das Molverhältnis tertiäres Amin : Kobaltverbindung beträgt vorzugsweise 2 : 1 bis 50 : 1.

Bei der Hydrocarboxyalkylierung wird ein Druck von 200 000 bis 350 000 hPa bevorzugt. Die Reaktion wird im allgemeinen in einem Autoklaven durchgeführt.

In einer besonderen Ausführungsform betrifft die Erfindung Verbindungen der allgemeinen Struktur

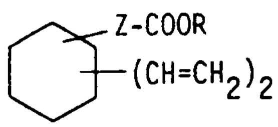

(II)

alleine oder im Gemisch miteinander, ihre Herstellung sowie ihre Verwendung.

Bei der Herstellung setzt man 1,2,4-Trivinylcyclohexan mit einem Alkohol mit 1 bis 10 C-Atomen und Kohlenmonoxid in Gegenwart eines Kobaltkatalysators und eines tertiären Amins bei 120 bis 140 °C und einem Druck von 150 000 bis 350 000 hPa in 6 bis 18 Stunden um.

Bei dieser Reaktion wird ein Produktgemisch erhalten, das im allgemeinen Verbindungen der folgenden Strukturen aufweist:

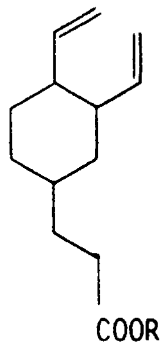
(V)

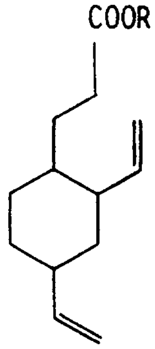
(VI)

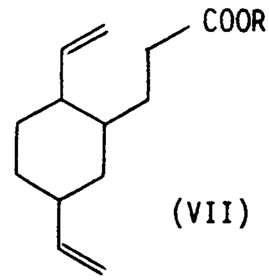
(VII)

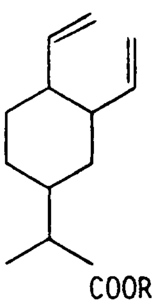
(VIII)

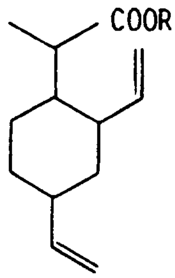
(IX)

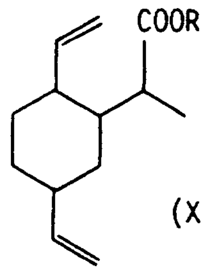
(X)

In einer weiteren besonderen Ausführungsform betrifft die Erfindung Verbindungen der allgemeinen Struktur

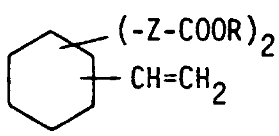
(III)

alleine oder im Gemisch miteinander, ihre Herstellung sowie ihre Verwendung.

Bei der Herstellung setzt man 1,2,4-Trivinylcyclohexan mit einem Alkohol mit 1 bis 10 C-Atomen und Kohlenmonoxid in Gegenwart eines Kobaltkatalysators und eines tertiären Amins bei 130 bis 160 °C und einem Druck von 150 000 bis 350 000 hPa in 12 bis 24 Stunden um.

Bei dieser Reaktion wird ein Produktgemisch erhalten, das im allgemeinen Verbindungen der folgenden Strukturen aufweist:

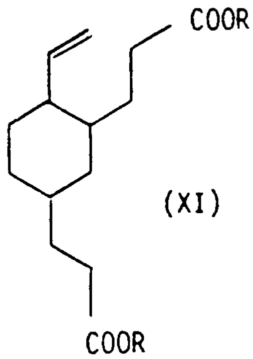
(XI)

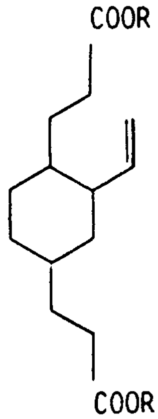
(XII)

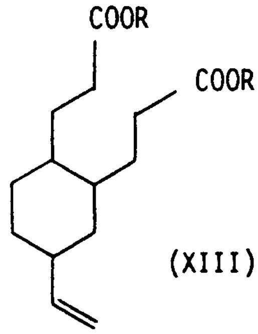
(XIII)

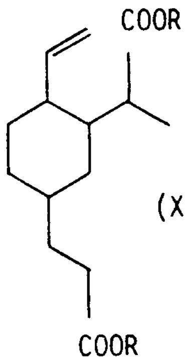
(XIV)

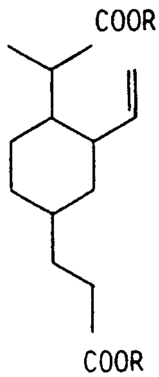
(XV)

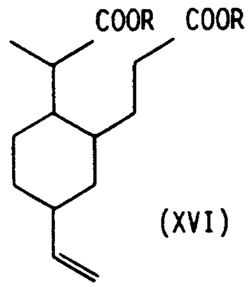
(XVI)

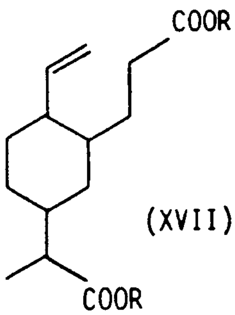
(XVII)

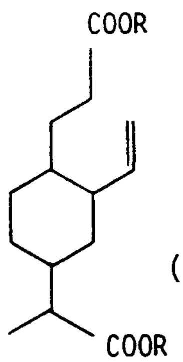
(XVIII)

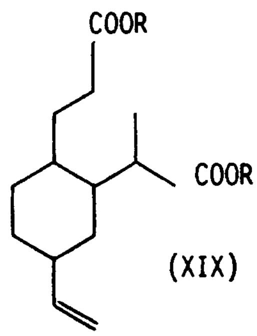
(XIX)

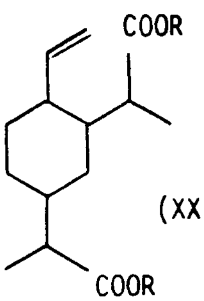
(XX)

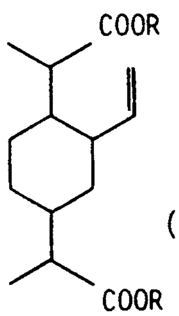
(XXI)

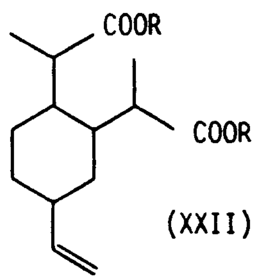
(XXII)

In einer dritten besonderen Ausführungsform betrifft die Erfindung Verbindungen der allgemeinen Struktur

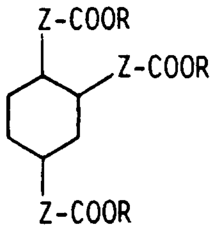

(IV)

alleine oder im Gemisch miteinander, ihre Herstellung sowie ihre Verwendung.

Bei der Herstellung dieser Verbindungen setzt man 1,2,4-Trivinylcyclohexan mit einem Alkohol mit 1 bis 10 C-Atomen und Kohlenmonoxid in Gegenwart eines Kobaltkatalysators und eines tertiären Amins bei 130 bis 170 °C und einem Druck von 150 000 bis 350 000 hPa in 36 bis 48 Stunden um.

Bei dieser Reaktion wird ein Produktgemisch erhalten, das im allgemeinen Verbindungen der folgenden Strukturen aufweist:

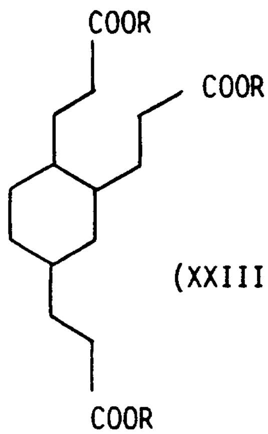

(XXIII)

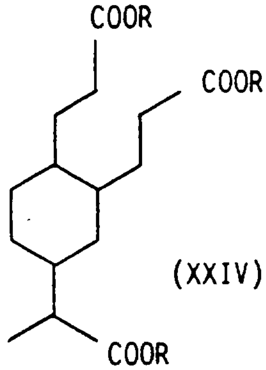

(XXIV)

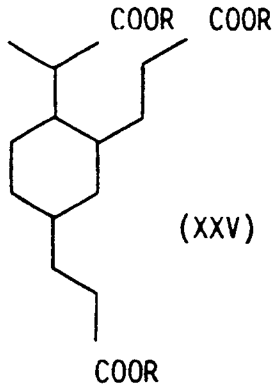

(XXV)

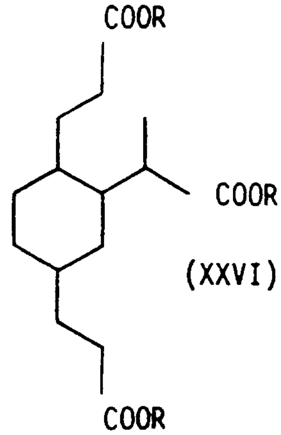

(XXVI)

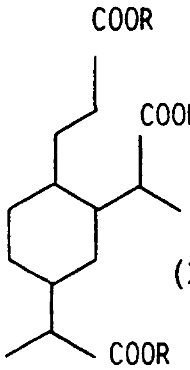

(XXVII)

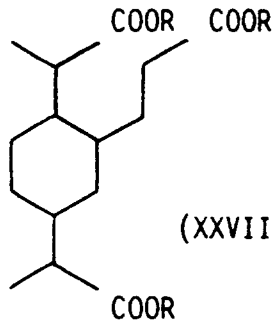

(XXVIII)

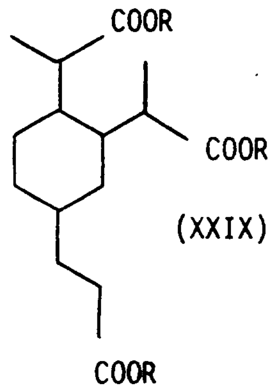

(XXIX)

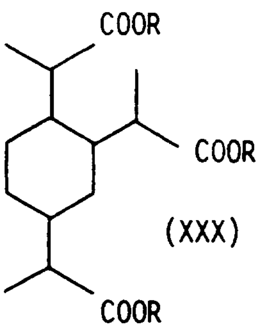

(XXX)

Das erfindungsgemäße Verfahren liefert die carboxylgruppenhaltigen Cyclohexanderivate allgemein bei hohen Ausbeuten mit hoher Selektivität.

Die Verbindungen der Struktur II erhält man mit einer Selektivität von über 75 %. Die Verbindungen der Struktur III gewinnt man bei einem Umsatz von über 90 % mit einer Selektivität von über 55 %. Das erfindungsgemäße Herstellverfahren liefert außerdem die Verbindungen der Struktur IV bei einem Umsatz von über 95 % mit einer Selektivität von über 75 %.

Die carboxylgruppenhaltigen Cyclohexanderivate können alleine oder im Gemisch miteinander als Monomere oder als Comonomere bei Polymerisationen und Polykondensationen eingesetzt werden.

Bei der Polymerisation werden Monomere, wie Ethen, Propen, Isobuten, 1-Buten, 1-Penten, Isopren oder Butadien, vorzugsweise mitverwendet.

Andere geeignete Monomere sind beispielsweise Acrylsäure, Acrylsäureester, Acrylsäurenitril, Acrylsäureamide, Methacrylsäure, Methacrylsäureester, Methacrylsäureamide, Maleinsäureanhydrid, Maleinsäurehalb- oder -vollester, Maleinsäureamide, Maleinsäureimide, Vinylacetat, Acrylnitril, Methacrylnitril, Styrol, alpha-Methylstyrol, Vinylchlorid, Vinylfluorid oder Vinylidenfluorid.

Die carboxylgruppenhaltigen Cyclohexanderivate können alleine oder im Gemisch miteinander als Monomere bei Polykondensationen eingesetzt werden. Man kann sie zur Herstellung von Alkydharzen, Polyestern oder Polyamiden verwenden. Sie können Bestandteile von Epoxidharzen sein oder Estergruppen

enthaltende Schmierstoffe verbessern.

Beispiele

In den Beispielen, die die Erfindung verdeutlichen sollen, wird ein 5-l-VA-Stahlautoklav verwendet.

Die Reaktionskomponenten 1,2,4-Trivinylcyclohexan, Alkohol, Kobalt(II)salz und 4-Picolin werden eingefüllt und auf Reaktionstemperatur gebracht. Dann wird Wasserstoff aufgedrückt, worauf durch Aufdrücken von Kohlenmonoxid der Gesamtdruck aufgebaut wird. Der Druckabfall während der Reaktion wird durch ständige Kohlenmonoxid-Zufuhr ausgeglichen.

Nach beendeter Reaktion wird auf Raumtemperatur abgekühlt und entspannt. Die gaschromatographisch bestimmten Ausbeuten werden in Mol-%, bezogen auf eingesetztes 1,2,4-Trivinylcyclohexan, angegeben.

Beispiel 1

Einwaage:

908,9 g ( 5,6 Mol) 1,2,4-Trivinylcyclohexan
717,7 g (22,4 Mol) Methanol
62,6 g ( 0,672 Mol) 4-Picolin
99,0 g ( 0,168 Mol) Co(II)naphthenat (10%ig an Co)

Reaktionsbedingungen:

$$p_{H_2} = 10\,000 \text{ hPa (anfangs)}; \; p_{ges} = 280\,000 \text{ hPa}$$

Reaktionstemperatur: 130 °C; Reaktionszeit: 12 h

Man erhält in 77,0%iger Selektivität Verbindungen der Struktur II. Der Rest enthält isomerisiertes Ausgangsprodukt und Verbindungen der Struktur III.

Bei der fraktionierten Destillation geht bei 62 bis 70 °C und 0,03 hPa das Gemisch über.

Beispiel 2

Einwaage:

762,8 g 1,2,4-Trivinylcyclohexan
903,5 g Methanol
52,5 g 4-Picolin
83,1 g Co(II)naphthenat (10%ig an Co)

Reaktionsbedingungen:

$$p_{H_2} = 10\,000 \text{ hPa (anfangs)}; \; p_{ges} = 280\,000 \text{ hPa}$$

Reaktionstemperatur: 130 °C; Reaktionszeit: 12 h

Man erhält Verbindungen der Struktur II in 81,1%iger Selektivität.

Beispiel 3

Einwaage:

762,8 g 1,2,4-Trivinylcyclohexan
903,5 g Methanol
52,5 g 4-Picolin
83,1 g Co(II)naphthenat (10%ig an Co)

Reaktionsbedingungen:

$$p_{H_2} = 5\,000 \text{ hPa (anfangs)};\ p_{ges} = 280\,000 \text{ hPa}$$

Reaktionstemperatur: 130 °C; Reaktionszeit: 12 h

Man erhält Verbindungen der Struktur II in 78,0%iger Selektivität.

Beispiel 4

Einwaage:

990,0 g ( 6,1 Mol) 1,2,4-Trivinylcyclohexan
625,4 g (19,5 Mol) Methanol
68,2 g ( 0,73 Mol) 4-Picolin
107,8 g ( 0,183 Mol) Co(II)naphthenat (10%ig an Co)

Reaktionsbedingungen:

$$p_{H_2} = 5\,000 \text{ hPa (anfangs)};\ p_{ges} = 280\,000 \text{ hPa}$$

Reaktionstemperatur: 130 °C; Reaktionszeit: 24 h

Man erhält bei einem Umsatz von 90,6 % in 59,3%iger Selektivität Verbindungen der Struktur III. Der Rest enthält isomerisiertes Ausgangsprodukt, Mono-Hydrocarboxymethylierungsprodukt und Tri-Hydrocarboxymethylierungsprodukt.

Bei der fraktionierten Destillation geht bei 103 bis 118 °C und 0,03 hPa das Gemisch über.

Beispiel 5

Einwaage:

990,0 g 1,2,4-Trivinylcyclohexan
625,4 g Methanol
68,2 g 4-Picolin
107,8 g Co(II)naphthenat (10%ig an Co)

Reaktionsbedingungen:

$$p_{H_2} = 5\,000 \text{ hPa (anfangs)};\ p_{ges} = 280\,000 \text{ hPa}$$

1. Stufe 130 °C/12 h

2. Stufe 140 °C/12 h
Man erhält bei einem Umsatz von über 98 % in 67,1%iger Selektivität Verbindungen der Struktur III.

Beispiel 6

Einwaage:

990,0 g 1,2,4-Trivinylcyclohexan
625,4 g Methanol
68,2 g 4-Picolin
107,8 g Co(II)naphthenat (10%ig an Co)

Reaktionsbedingungen:

$$p_{H_2} = 10\,000 \text{ hPa (anfangs)}; \; p_{ges} = 280\,000 \text{ hPa}$$

Reaktionstemperatur: 150 °C; Reaktionszeit: 12 h
Man erhält bei einem Umsatz von über 99 % in 62,0%iger Selektivität Verbindungen der Struktur III.

Beispiel 7

Einwaage:

990,0 g 1,2,4-Trivinylcyclohexan
625,4 g Methanol
68,2 g 4-Picolin
107,8 g Co(II)naphthenat (10%ig an Co)

Reaktionsbedingungen:

$$p_{H_2} = 10\,000 \text{ hPa (anfangs)}; \; p_{ges} = 280\,000 \text{ hPa}$$

Reaktionstemperatur: 160 °C; Reaktionszeit: 12 h
Man erhält bei einem Umsatz von über 99 % in 59,0%iger Selektivität Verbindungen der Struktur III.

Beispiel 8

Einwaage:

1 136,1 g 1,2,4-Trivinylcyclohexan
471,0 g Methanol
78,2 g 4-Picolin
123,8 g Co(II)naphthenat (10%ig an Co)

Reaktionsbedingungen:

$$p_{H_2} = 10\,000 \text{ hPa (anfangs)}; \; p_{ges} = 280\,000 \text{ hPa}$$

Reaktionstemperatur: 140 °C; Reaktionszeit: 12 h

Man erhält bei einem Umsatz von über 96 % in 57,0%iger Selektivität Verbindungen der Struktur III.

Beispiel 9

Einwaage:

990,0 g ( 6,1 Mol) 1,2,4-Trivinylcyclohexan
625,4 g (19,5 Mol) Methanol
68,2 g ( 0,73 Mol) 4-Picolin
107,8 g ( 0,183 Mol) Co(II)naphthenat (10%ig an Co)

Reaktionsbedingungen:

$$p_{H_2} = 10\,000 \text{ hPa (anfangs)}; \; p_{ges} = 280\,000 \text{ hPa}$$

1. Stufe 130 °C/24 h
2. Stufe 160 °C/24 h

Man erhält bei einem Umsatz von über 99 % in 78,4%iger Selektivität Verbindungen der Struktur IV. Der Rest enthält Di-Hydrocarboxymethylierungsproduktund Hochsieder.

Bei der fraktionierten Destillation geht bei 145 bis 158 °C und 0,04 hPa das Gemisch über.

Beispiel 10

Einwaage:

990,0 g 1,2,4-Trivinylcyclohexan
625,4 g Methanol
68,2 g 4-Picolin
107,8 g Co(II)naphthenat (10%ig an Co)

Reaktionsbedingungen:

$$p_{H_2} = 10\,000 \text{ hPa (anfangs)}; \; p_{ges} = 280\,000 \text{ hPa}$$

1. Stufe 130 °C/24 h
2. Stufe 170 °C/24 h

Man erhält bei einem Umsatz von über 99 % in 80,0%iger Selektivität Verbindungen der Struktur IV.

Beispiel 11

Einwaage:

973,8 g 1,2,4-Trivinylcyclohexan
595,9 g Methanol
89,4 g 4-Picolin
141,8 g Co(II)naphthenat (10%ig an Co)

Reaktionsbedingungen:

$$p_{H_2} = 10\,000 \text{ hPa (anfangs)}; \; p_{ges} = 280\,000 \text{ hPa}$$

1. Stufe 130 °C/24 h
2. Stufe 170 °C/24 h

Man erhält bei einem Umsatz von über 99 % in 81,8%iger Selektivität Verbindungen der Struktur IV.

<u>Beispiel 12</u>

Einwaage:

1 006,3 g 1,2,4-Trivinylcyclohexan
615,8 g Methanol
69,3 g 4-Picolin
109,6 g Co(II)naphthenat (10%ig an Co)

Reaktionsbedingungen:

$$p_{H_2} = 10\,000 \text{ hPa (anfangs)}; \; p_{ges} = 280\,000 \text{ hPa}$$

1. Stufe 140 °C/24 h
2. Stufe 170 °C/24 h

Man erhält bei einem Umsatz von über 99 % in 77,0%iger Selektivität Verbindungen der Struktur IV.

**Patentansprüche**

**1.** Verbindungen der allgemeinen Struktur

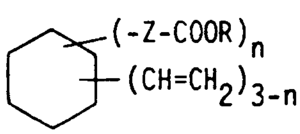

(I)

alleine oder im Gemisch miteinander, dadurch gekennzeichnet, daß die Substituenten am Cyclohexanring in 1-, 2- und 4-Position stehen, Z eine $-CH_2-CH_2-$Gruppe oder eine

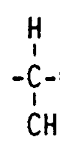-Gruppe,

R eine Alkylgruppe mit 1 bis 10 C-Atomen und n eine Zahl von 1 bis 3 ist.

2. Verbindungen nach Anspruch 1 der Struktur

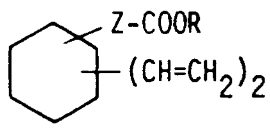

(II)

alleine oder im Gemisch miteinander.

3. Verbindungen nach Anspruch 1 der Struktur

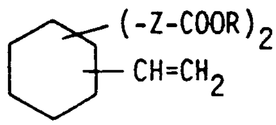

(III)

alleine oder im Gemisch miteinander.

4. Verbindungen nach Anspruch 1 der Struktur

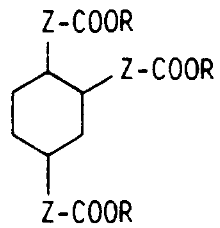

(IV)

alleine oder im Gemisch miteinander.

5. Verfahren zur Herstellung der Verbindungen von Anspruch 1,
dadurch gekennzeichnet,
daß man 1,2,4-Trivinylcyclohexan mit einem Alkohol mit 1 bis 10 C-Atomen und Kohlenmonoxid in Gegenwart eines Kobaltkatalysators und eines tertiären Amins bei 120 bis 170 °C und einem Druck von 150 000 bis 350 000 hPa in 6 bis 48 Stunden umsetzt.

6. Verfahren zur Herstellung der Verbindungen von Anspruch 2,
dadurch gekennzeichnet,
daß man 1,2,4-Trivinylcyclohexan mit einem Alkohol mit 1 bis 10 C-Atomen und Kohlenmonoxid in Gegenwart eines Kobaltkatalysators und eines tertiären Amins bei 120 bis 140 °C und einem Druck von 150 000 bis 350 000 hPa in 6 bis 18 Stunden umsetzt.

7. Verfahren zur Herstellung der Verbindungen von Anspruch 3,
dadurch gekennzeichnet,
daß man 1,2,4-Trivinylcyclohexan mit einem Alkohol mit 1 bis 10 C-Atomen und Kohlenmonoxid in Gegenwart eines Kobaltkatalysators und eines tertiären Amins bei 130 bis 160 °C und einem Druck von 150 000 bis 350 000 hPa in 12 bis 24 Stunden umsetzt.

8. Verfahren zur Herstellung der Verbindungen von Anspruch 4,
dadurch gekennzeichnet,
daß man 1,2,4-Trivinylcyclohexan mit einem Alkohol mit 1 bis 10 C-Atomen und Kohlenmonoxid in Gegenwart eines Kobaltkatalysators und eines tertiären Amins bei 130 bis 170 °C und einem Druck von 150 000 bis 350 000 hPa in 36 bis 48 Stunden umsetzt.

9. Verwendung der Verbindungen von Anspruch 1 als Monomere und Comonomere bei Polymerisationen und Polykondensationen.

**Claims**

1. Compounds of the general structure

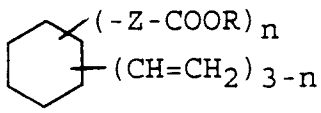

(I) ,

alone or in admixture, characterised in that the substituents on the cyclohexane ring are in the 1-, 2- and 4-positions, Z is a $-CH_2-CH_2-$ group or a

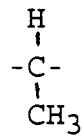

group, R is an alkyl group having from 1 to 10 carbon atoms and n is a number from 1 to 3.

2. Compounds according to claim 1 of the structure

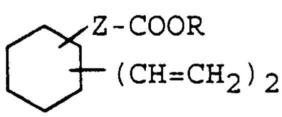

(II) ,

alone or in admixture.

3. Compounds according to claim 1 of the structure

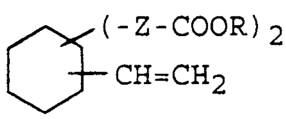

(III) ,

alone or in admixture.

4. Compounds according to claim 1 of the structure

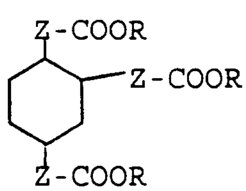

(IV),

alone or in admixture .

5. A process for preparing the compounds of claim 1, characterized in that 1,2,4-trivinylcyclohexane is reacted with an alcohol having from 1 to 10 carbon atoms and carbon monoxide in the presence of a

cobalt catalyst and a tertiary amine at from 120 to 170 °C and a pressure of from 150,000 to 350,000 hPa in from 6 to 48 hours.

6. A process for preparing the compounds of claim 2, characterised in that 1,2,4-trivinylcyclohexane is reacted with an alcohol having from 1 to 10 carbon atoms and carbon monoxide in the presence of a cobalt catalyst and a tertiary amine at from 120 to 140 °C and a pressure of from 150,000 to 350,000 hPa in from 6 to 18 hours.

7. A process for preparing the compounds of claim 3, characterised in that 1,2,4-trivinylcyclohexane is reacted with an alcohol having from 1 to 10 carbon atoms and carbon monoxide in the presence of a cobalt catalyst and a tertiary amine at from 130 to 160 °C and a pressure of from 150,000 to 350,000 hPa in from 12 to 24 hours.

8. A process for preparing the compounds of claim 4, characterised in that 1,2,4-trivinylcyclohexane is reacted with an alcohol having from 1 to 10 carbon atoms and carbon monoxide in the presence of a cobalt catalyst and a tertiary amine at from 130 to 170 °C and a pressure of from 150,000 to 350,000 hPa in from 36 to 48 hours.

9. Use of the compounds of claim 1 as monomers or comonomers in polymerizations and polycondensations.

**Revendications**

1. Les composés de la structure générale

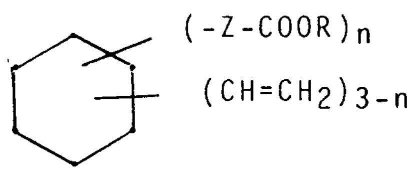

(I),

seuls ou en mélange entre eux,
caractérisés par le fait
que les substituants sur l'anneau cyclo-hexane se trouvent en position 1, 2 et 4,
que Z représente un groupe $CH_2$-$CH_2$ ou un groupe

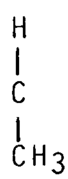

,

que R représente un groupe alkyle comportant de 1 à 10 atomes de carbone, et
que n est un nombre d'une valeur de 1 à 3.

2. Les composés selon la revendication 1, de structure

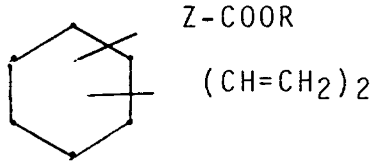

(II),

seuls ou en mélange entre eux,

3. Les composés selon la revendication 1, de structure

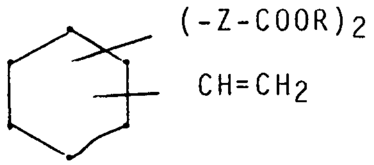

(III),

seuls ou en mélange entre eux.

4. Les composés selon la revendication 1, de structure

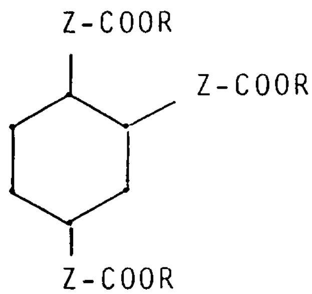

(IV),

seuls ou en mélange entre eux.

5. Procédé de préparation des composés selon la revendication 1, caractérisé en ce que l'on fait réagir à une température de 120 à 170 °C et sous une pression de 150 000 à 350 000 hPa, en 6 à 48 heures, du 1,2,4-trivinyl-cyclohexane sur un alcool comportant de 1 à 10 atomes de carbone et sur du monoxyde de carbone, en présence d'un catalyseur au cobalt et d'une amine tertiaire.

6. Procédé de préparation des composés selon la revendication 2, caractérisé en ce que l'on fait réagir à une température de 120 à 140 °C et sous une pression de 150 000 à 350 000 hPa, en 6 à 18 heures, du 1,2,4-trivinyl-cyclohexane sur un alcool comportant de 1 à 10 atomes de carbone et sur du monoxyde de carbone, en présence d'un catalyseur au cobalt et d'une amine tertiaire.

7. Procédé de préparation des composés selon la revendication 3, caractérisé en ce que l'on fait réagir à une température de 130 à 160 °C et sous une pression de 150 000 à 350 000 hPa, en 12 à 24 heures, du 1,2,4-trivinyl-cyclohexane sur un alcool comportant de 1 à 10 atomes de carbone et sur du monoxyde de carbone, en présence d'un catalyseur au cobalt et d'une amine tertiaire.

8. Procédé de préparation des composés selon la revendication 4, caractérisé en ce que l'on fait réagir à une température de 130 à 170 °C et sous une pression de 150 000 à 350 000 hPa, en 36 à 48 heures, du 1,2,4-trivinyl-cyclohexane sur un alcool comportant de 1 à 10 atomes de carbone et sur du monoxyde de carbone, en présence d'un catalyseur au cobalt et d'une amine tertiaire.

9. L'utilisation des composés de la revendication 1 en tant que monomères et comonomères dans des polymérisations et des polycondensations.